(19) Europäisches Patentamt
European Patent Office
Office européen des brevets

(11) **EP 2 024 893 B1**

(12) **EUROPEAN PATENT SPECIFICATION**

(45) Date of publication and mention of the grant of the patent:
**04.03.2015 Bulletin 2015/10**

(51) Int Cl.:
*G06F 19/00* (2011.01)      *G06T 7/40* (2006.01)
*G01N 33/543* (2006.01)

(21) Application number: **07794520.2**

(22) Date of filing: **03.05.2007**

(86) International application number:
**PCT/US2007/010727**

(87) International publication number:
**WO 2007/133465 (22.11.2007 Gazette 2007/47)**

(54) **A LASER ILLUMINATION SYSTEM IN FLUORESCENT MICROSCOPY**

LASERBELEUCHTUNGSSYSTEM IN EINEM FLUORESZENZMIKROSKOP

SYSTÈME D'ÉCLAIRAGE LASER EN MICROSCOPIE FLUORESCENTE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.05.2006 US 800258 P**

(43) Date of publication of application:
**18.02.2009 Bulletin 2009/08**

(73) Proprietor: **Janssen Diagnostics, LLC Raritan, NJ 08869 (US)**

(72) Inventors:
• **GREVE, Jan**
  **7500 AE Enschede (NL)**
• **SCHREUDER, Frederik**
  **7500 AE Enschede (NL)**

(74) Representative: **Goodfellow, Hugh Robin Carpmaels & Ransford LLP One Southampton Row London WC1B 5HA (GB)**

(56) References cited:
WO-A1-00/32293          GB-A- 2 416 444
US-A- 5 432 054          US-A- 6 151 405
US-A1- 2006 234 269

• HOFFNAGLE J A ET AL: "DESIGN AND PERFORMANCE OF A REFRACTIVE OPTICAL SYSTEM THAT CONVERTSA GAUSSIAN TO A FLATTOP BEAM", APPLIED OPTICS, OPTICAL SOCIETY OF AMERICA, WASHINGTON, DC; US, vol. 39, no. 30, 20 October 2000 (2000-10-20), pages 5488-5499, XP000981156, ISSN: 0003-6935, DOI: 10.1364/AO.39.005488
• RACILA ET AL.: 'Detection and Characterization of Carcinoma Cells in the Blood' PROC. NATL. ACAD. SCI. USA vol. 95, April 1998, pages 4589 - 4594, XP002132943
• TIBBE ET AL.: 'Optical tracking and Detection of Immunomagnetically Selected at Aligned Cells' NATURE BIOTECHNOLOGY vol. 17, December 1999, pages 1210 - 1213, XP002327805
• Reinhard Völkel ET AL: "Homogenisierung von laserstrahlen", Photonik, 13 June 2006 (2006-06-13), pages 76-79, XP055089572, Retrieved from the Internet: URL:chrome://ietab2/content/reloaded.html? url=http://www.suss-microoptics.com/media/downloads/publications/Homogenisierung_von_Laserstrahlen.pdf [retrieved on 2013-11-21]

EP 2 024 893 B1

**Description**

**Technical Field**

This invention relates generally to devices and methods to obtain to scan and obtain images of objects, and more particularly to images reconstructed from partial sub-images of object such as cells obtained from biological fluids that are distributed in a two-dimensional plane. More specifically, the present invention is applicable in fluorescent microscopy and flow cytometry where the illumination.

**Background of the Invention**

The identification and enumeration of circulating carcinoma cells of epithelial origin in the blood of cancer patients that maybe present at frequencies of less than one carcinoma cell per ml of blood has been detected using magnetic enrichment and image cytometry as in, for example, CellTracks® Systems (Immunicon Corporation, Huntingdon Valley, PA). Using a combination of epithelial cell enrichment by magnetic means in combination with analysis by multi-parametric flow cytometry, significant differences in the number of "circulating tumor cells" were found between healthy individuals and patients with breast cancer (Racila et al., Proc. Nat. Acad. Sci. 95, 4589-4594, 1998). In several studies, such "circulating tumor cells" (CTC) were defined as events expressing the following characteristics: positive for the epithelial cell marker cytokeratin, negative for the leukocyte marker CD45, positive staining with a nucleic acid dye, and light scattering properties that are compatible with cells. However, morphometric confirmations of the detected events as cells and further molecular evidence is lacking in flow cytometric methods, but is clearly needed to assure that the detected rare events are indeed tumor cells derived from a primary tumor. Automated image analysis systems have been introduced to reduce subjective errors in cell classification between different operators in manual methods, but such prior art systems without preliminary cell enrichment steps still inherently lack sensitivity. Several automated cell imaging systems have been described or are commercially available for cell analysis. The system developed by Chromavision, ACIS™ or Automated Cellular Imaging System (Douglass et al., US patent 6,151,405) uses colorimetric pattern recognition by microscopic examination of prepared cells by size, shape, hue and staining intensity as observed by an automated computer controlled microscope and/or by visual examination by a health care professional. The system uses examination of cells on microscope slides and was designed for tissue sections. The SlideScan™ or MDS™ systems of Applied Imaging Corp. (Saunders et al., US patent 5,432,054) is described as an automated, intelligent microscope and imaging system that detects cells or "objects" by color, intensity, size, pattern and shape followed by visual identification and classification. In contrast to the ACIS system this system has the ability to detect fluorescent labels which provides more capability. However, these and other currently available methodologies are not sufficiently sensitive for accurate classification and typing of rare events such as circulating tumor cells in blood.

Thus, an imaging system (US 10/612,144) was developed to detect fluorescent signals at a relatively high speed, performing similar to standard flowcytometry. The system is based on the separation of cells by first labeling with immunomagnetic particles. These particles are colloidal paramagnetic particles linked to a monoclonal antibody specific for a characteristic cell surface antigen found on the target cell. When sufficiently bound to the target population of cells, a magnetic field forces the immunomagnetic particle-cell complex to rise to the top of the chamber where the cells align between nickel lines. When targeting epithelial-derived cells with a monoclonal antibody to the Epithelial Cell Adhesion molecule (EpCAM), erythrocytes and other non-labeled cells separate from the labeled cells which rise up to the upper glass surface of the chamber. Since this is in a known volume, enumeration of cells can easily be determined. A red diode laser (635 nm) is focused as an elliptical beam on the chamber, in conjunction with the nickel lines. The reflection from the nickel lines is used as a signal for the tracking and focusing of the laser beam on the aligned cells. The sample chamber is moved with the X-Y stage in the Y direction and the laser is scanning one line at a time. The aligned cells are illuminated one by one and the emitted fluorescence is measured with photomultipliers. The advantages of this design are that only the target cells align on the inner surface of the viewing portion of the chamber and the aligned cells are easily relocated after the first analysis has been completed. Both benefits ensure that extremely small populations of target cells can be measured with the imaging system described in US 10/612,144 after magnetic enrichment. Scatter plots from fluorescent data, generated from multi-labeling experiments, can be used to select rare events. With the cells aligned along the inner surface of the viewing portion of the chamber, fluid underneath the cells can be replaced, and the cells can be further analyzed.

Prior technology has been based upon a standard full frame charge coupled device (CCD) camera with, for example, a frame rate of 25 frames per second. The cell is scanned with a moving stage or a scanning mirror. The images acquired with the camera are added together, forming a complete image of the cell. The disadvantage is that when scanning, the laser is only illuminating a small area of the cell. Therefore it is necessary to scan the object (or cell) to image the entire cell. To acquire enough photons and to prevent bluring due to the movement of the cell it is necessary to scan slowly. Another disadvantage of this type of imaging is that a full frame camera is not collecting photons during readout of the CCD. Consequently, all fluorescent photons emitted at that moment are useless. The camera is closed for readout for about 16% of the total time for a pixel area of 512x512 pixels at 25 fps and a fast readout rate of 40 Mhz.

For Fluorescent microscopy and Flow Cytometry, illumination with a laser is in most cases done with the use of Gaussian beam profile. From the Gaussian profile only a small portion of the beam has a homogeneous illumination which is useful in imaging.

Accordingly, the present invention seeks to improve upon traditional CCD technology as related to image cytometric analysis, such as CellSpotter™ Systems, and to permit detection, enumeration and accurate classification of rare target species, such as CTC in blood or other fluids.

WO 00/32293 discloses apparatuses and methods for separating, immobilizing, and quantifying biological substances from within a fluid medium. Biological substances are observed by employing a vessel with a chamber therein, the vessel comprising a transparent collection wall with a high internal gradient magnetic capture structure on the wall. Magnets create an externally-applied force for transporting magnetically responsive material toward the transparent collection wall. The magnetic capture structure comprises a plurality of ferromagnetic members and has a uniform or non-nonuniform spacing between adjacent members.

## Summary of the Invention

This invention improves upon the devices and methods that permit the application of novel imaging capabilities to such systems as the CellTracks® analysis system as described by Tibbe et al. (Nature Biotech. 17, 1210-13, 1999). The devices and methods described by Tibbe and in the disclosure of this invention can also be applied to other target objects. However, the primary application is rapid immunomagnetic selection of rare cells from blood followed by automated alignment of the isolated cells and automated image analysis

The present invention provides a novel scanning and imaging diagnostic system for detection, classification and enumeration of cells, which comprises an efficient automated means for collecting and aligning immunomagnetically labeled target cells from body fluids, and in which such collected cells also bear at least one immuno-specific fluorescent label that differentiates target from non-target cells labeled with different fluorescent label(s).

In accordance with the present invention, the new homogeneous illumination and imaging techniques can be integrated into a system such as the imaging system in US 10/612,144 to obtain high quality fluorescence images. The discoveries described and claimed herein have greatly improved the detection, enumeration and classification of rare cells over systems and methods in prior art. Efficient detection of cells at very low frequencies, so called rare events, requires minimal sample handling to avoid losses of cells. Furthermore, the volume from which the rare cells are separated and enriched should be as large as possible to increase the sensitivity of detection. With the development and application of the disclosed novel techniques, fluorescent images of specific events can now be obtained resulting in a highly accurate identification, thus making the inventive system a powerful tool for the detection of rare events in body fluids.

## Brief Description of the Drawings

Figure 1
Depicts the position of the homogenizer in the Time-Delay-Integration System discussed in U.S. 11/344,757 with the incorporation of the beam homogenizer with rotating diffuser and microlens array.

Figure 2
Diagram of a beam homogenizer (Suss-MicroOptics, Neuchatel,Switserland) consisting of a pair of micro lens arrays in combination with a rotating diffuser, creating a square homogeneous profile.

Figure 3
Image of a homogenous layer using Acradine Orange. (a) Coherence length reduced with a rotating diffuser. (b) Periodic structure in the Fourier plane created due to interference of coherent light source, without rotating diffuser. (c) Illumination profile perpendicular to the scanning direction.

Figure 4
A flattop profile created by quadratic lens apertures and overlaid in the Fourier plane of the microscope objective, resulting in an illumination area matching the detection area of the CCD.

**DETAILED DESCRIPTION OF THE INVENTION**

The present invention combines fluorescent microscopy and a beam homogenizer to illuminate the sample with a laser. The beam homogenizer is particular useful for automated fluorescent microscopy.

The illumination area can be adapted to a small area that exactly matches the size of the detection area (CCD), without unnecessary bleaching of the sample. (Only the collection area is illuminated)

The homogeneity of the illumination with this homogenizer is also better than other methods like HBO illumination. The homogenizer can be used for the entire visible wavelength range (300 - 850 nm). Compared with the HBO a much higher power density can be achieved while the beam homogenizer uses a laser. Another advantage of this system is that the beam profile is not so sensitive to small variations in the positional alignment of the laser. In flow cytometry a small part of a Gaussian profile is used to obtain a homogeneous illumination, therefore most of the illumination power is not used. With the beam homogenizer almost all the light is used and not wasted. The present invention provides a homogeneous profile and a laser alignment process that is not as sensitive to small deviations.

Racila et al. (Proc. Nat. Acad. Sci. 95, 4589-94, 1998) described a method for separating breast carcinoma cells from blood using a sequence of steps including immunomagnetic labeling followed by immunophenotyping analysis in a flow cytometer. As stated in US 10/612,144, this imaging system is capable of immunophenotyping individual cells and confirming their identity by providing a fluorescence image. The procedure was tested by the detection of cultured cells of the breast cancer cell line, SKBR3, spiked into whole blood.

The imaging system, disclosed in US 10/612,144, consists of a standard monochrome surveillance charge-coupled device (CCD) camera at a frame rate 25Hz, having manual gain adjustment (US 10/612,144). Using this arrangement with the insertion of a removable mirror into the light beam, the fluorescent light captured by the compact disk (CD) objective is focused by the spherical achromatic lens (f=150 mm) onto the CCD instead of a simple pinhole. The CD objective consists of a single aspherical lens, optimized to obtain a diffraction-limited spot size at a wavelength of 780 nm, similar to those used in current CD players. With a preferred numerical aperture (NA) of the lens is 0.45 and a lens diameter of 4 mm, the elliptical shape of the image is created by the two cylindrical lenses that are placed at a distance slightly larger than twice their individual focal lengths. The short axis of the elliptical focus is set at 4 $\mu$m (FWHM) which is smaller than the diameter of a cell, limiting the focus to one cell at any one time. The longer axis is larger than the Ni line spacing. The light focused on the Ni lines is reflected and used for feedback control. The Ni lines are present on a 0.5 mm thick glass substrate. Focusing the laser light (635nm) onto the Ni lines through the glass substrate with the CD-objective results in a non-homogeneous laser focus.

The imaging system is based on magnetic collection methods that do not stress or distort the cells as is commonly observed in cytospin systems using centrifugal deposition on slides. Hence, the magnetically aligned cells maintain their native three-dimensional shape and volume. The device and method described herein can also be used to obtain a confocal image of cells and, therefore, to enhance the image quality by allowing a more accurate determination of the 3D-distribution of the fluorescent dye inside the cells.

A full image of a measured event, wherein a full image is defined as a reconstructed image consisting of combined multiple digitized sub-images, is obtained by the acquisition of positional information. Before image acquisition of a previously measured event, the event must be relocated on the sample chamber. Consequently, spatial information, such as x-y sample coordinates for each sub-image, is needed. To obtain positional information in the y-direction, the stage which moves both the magnets and the sample chamber has been equipped with an encoder that has a resolution of 0.2 microns. The line number for a sub-image of a specific event is measured to give positional information in the x-direction. The encoder signals, together with the line number, are stored in memory and are coupled to the signals measured from the photomultiplier tube (PMT) for each sub-image. In this manner, all the measured sub-image events are associated with and indexed to an x-y position for the sample.

To return to the position where a specific event or sub-image was measured, the laser focus shifts by the number of lines equal to the current line number minus the line number on which the specific event was measured. Concurrently, the stage moves in the y-direction to the specific encoder position. An alternative method for obtaining encoder signals is to add profiles to the Ni lines to record position. This approach further permits the use of a significantly simpler and cheaper stage to move the sample. It should be further noted that while Ni lines are used to align the cells and to re-locate the cells using the line number on which the cell is measured with its corresponding encoder value the imaging invention described herein is not dependent on the presence of Ni lines or magnetic lines and can be used on any surface on which cells or other fluorescent objects of interest are present or can be deposited. Only the encoder data in the scan direction would be needed for reconstructing the image from the stored and grabbed sub-images.

The intensity profile at a particular position of the laser focus is homogeneous with the addition of the rotating diffuser and microlens array (SUSS-MicroOptics). Therefore, uniform illumination with a laser focus is provided through the use of a homogeneous intensity profile by scanning the laser across the cell surface with the movement of an optical component in the beam, as is done in a laser scanning microscope (Corle, TR, Confocal Scanning Microscopy and Related Imaging Systems, Academic Press, NY, 1995). In the imaging system of US 10/612,144, uniform illumination by this method would result in a loss of feedback, which in turn would result in a loss of positional information in the x-direction. The intensity profile is obtained after adding the individual pixel intensities of the focal spot image in the y-direction. With a Ni line spacing of 10 microns, the summed intensity profile has an intensity variation of $\pm$ 6 % across the line spacing. Moving a cell in the y-direction through this focus has the result that every part of the cell has received an almost equal illumination after it has passed through the laser focus. This method is used to obtain a full high quality fluorescent image of an aligned cell, based on summation of individual sub-images.

The magnets and chamber are positioned on a stage that moves the cells through the focus in the y-direction. To obtain a full image of a specific cell, the laser focus is shifted to the line where the specific sub-image event was measured and the stage is moved in the y-direction to the corresponding encoder position with a speed of 10 mm/sec. The stage is slowed down to a speed of 5 $\mu$m/sec when the distance to the cell position is 25 $\mu$m (25 microns).

A frame grabber card captures the CCD sub-images at 25 Hz and these are stored in memory. In each subsequent sub-image, a different part of the cell is illuminated since the laser focus in the scan direction is smaller than the cell diameter. Together with the sub-image capture, the encoder position is read and both are stored in computer memory. A total of 40 $\mu$m (40 microns) are scanned, corresponding to 200 captured sub-images each with 150 x 250 pixels. There is an average of a 0.2 $\mu$m (0.2 micron) shift with respect to each other. This corresponds to a shift of 2.63 pixels on the CCD surface. Using the encoder values, the captured sub-images are shifted over a number of pixels corresponding to the difference in their associated encoder values x 2.63, which are then summed or combined. The sub-image resolution in the y-direction is determined by the encoder resolution 0.2 $\mu$m (0.2 microns). The resolution in the x-direction is determined by the number of pixels in the image recorded in the x-direction 0.07 $\mu$m/pixel (0.07 microns/pixel). Both resolutions are smaller than the diffraction limit. Hence, it will be appreciated be those skilled in the art that the ultimate image resolution will not be determined by the encoder or the camera but by the imaging optics.

An integral component of the imaging optics in the present invention is the homogeneity of illumination throughout the image. Thus, a profile of this homogeneity was assessed with a thin layer of a concentrated fluorescent dye solution, placed in the same plane of the magnetically labeled cells such that they would be aligned between the Ni lines which are spaced 10 $\mu$m 10 microns apart. The dye layer was scanned and imaged at a speed of 5 $\mu$m/sec as previously described. For a uniform layer of fluorescent dye, one would expect a homogeneous fluorescent image, if the illumination were uniform.

Responsivity of a CCD is dependent upon the wavelength, expressed in Digital Numbers (DN). The Dalsa Eclipse line scanner has a high responsivity due to the high quantum efficiency in the visible region: This responsivity is typically 1950 DN/(nJ/cm$^2$) for a 0 dB gain in each column for the sensor.

Several noise sources are present in a CCD camera. The major sources are the readout noise, the photon noise, and dark current noise. These noises arise from the imaging array, the readout register, and the output structure. Photon noise is defined as photons incident on the CCD which are converted to photoelectrons within the silicon layer. These photoelectrons comprise the signal but also carry a statistical variation of fluctuations in the photon arrival rate at a given point. Readout noise is electronic noise which is inherent to the CCD. Readout noise refers to the uncertainty introduced during the process of quantifying the electronic signal on the CCD. Dark current noise is noise that arises from the statistical variation of thermally generated electrons. These electrons come from the CCD material through thermal vibration. All these noise sources contribute to the quality of the overall signal which is expressed as the signal to noise ration (SNR).

In the imaging system without TDI, SNR is improved by reducing the imaging scan speed, resulting in larger number of captured sub-images of a specific event and a better signal to noise ratio. However, no improvement in image quality for the APC labeled SKBR3 cells is observed when the image scanning speed was reduced. The limit of the imaging scan velocity is determined by the photo-bleaching rate of the dye molecules. No fluorescence is detected with the CCD camera if an SKBR3 cell is scanned for the second time, indicating that most of the APC molecules have already been photo-destroyed after the first scan. Reducing the scan speed would, therefore, make no difference in the detected fluorescence signal and would only result in increased fluorescent background. The optimal scan speed is, therefore, dependent on the individual dye characteristics and is different for each fluorescent dye. Therefore, to the improve signal to noise ratio it may be better to scan faster. On the other hand scanning faster than 5 microns/sec when using a camera with a frame rate of 25 Hz would result in a loss of resolution since the captured images would be spaced more than 0.2 microns apart. A CCD camera with higher frame rate would be needed to scan faster without losing resolution. Replacing the standard surveillance CCD camera with a more sensitive camera would also allow imaging of dimly stained cells.

The SNR in an imaging system with a TDI camera as described in the present invention depends upon the camera, the total integration time and the amount of signal photons hitting the CCD during integration. These signal photons come from fluorescent dyes that are attached to the cells. The total amount of fluorescence signal photons is not constant for these dyes, and is compounded by a reduced total time due to bleaching of the dyes. The result is that at slow scanning speeds (long integration times) most of the fluorescence gathered results in a high SNR. However, the scanning speed can be too slow where the number of photons emitted per second will be so small that the dark current dominates the charge distribution which results in a lower SNR.

Consequently, the scanning speed has to be adjusted to the particular dye used to obtain the maximum SNR. A typical situation would be to determine the optimum scanning speed for the sample stage in the Y-direction when a fluorescence indicator is used in order to obtain the highest SNR with the TDI imager of the present invention. The dependence of the SNR will be on the bleaching rate, the laser illumination and magnification.

At a low scanning velocity, the SNR improves with increasing velocity. The SNR also improves with higher illumination power. Maximum SNR is obtained when the total amount of photons is increased to more than the amount of dark current photons. Accordingly when scanning too slowly, the SNR will decrease while the rate of photon emitted by the fluorophores will not be high enough.

The original imaging system (US 10/612,144) used a red diode laser as an excitation source for several fluorescent labels. An encoder was used to give positional information of the cells and to relocate the aligned cells.

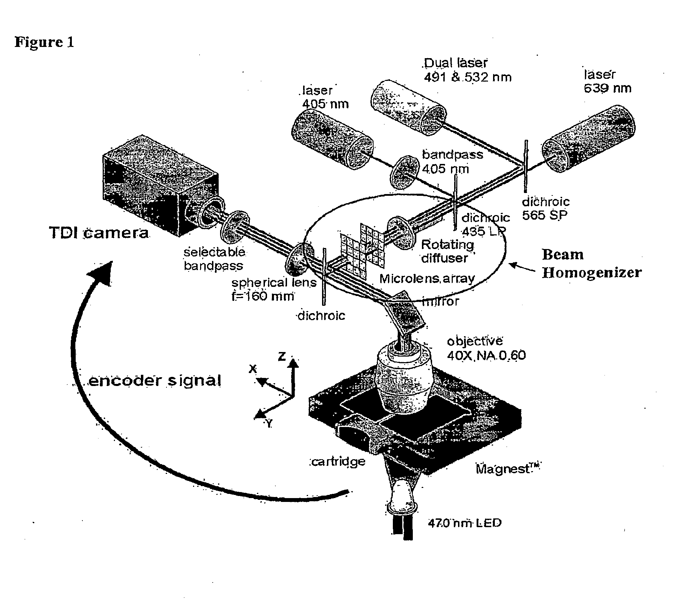

CellTracks with the TDI imager incorporates all red excitable dyes and the emissions of these dyes, along with a second green laser (532 nm) to extend the amount of dyes that can be used and to have an emission spectrum that is further apart (See Figure 1). This green laser beam is expanded 4 times with a beam expander. With a cylindrical lens, an elliptical spot is created which results in two focal planes after focusing with the CD objective. A dichroic mirror that reflects the green laser light and transmits the red laser light is used to combine the two laser lines. The green laser spot overlaps the spot of the red laser. An achromat replaces the CD lens to obtain a focal plane that is equal for the red and the green laser light in combination within the beam. This allows the beams to run in parallel. A mirror/dichroic combination projects the red fluorescence at a different position on the CCD which is followed by the green fluorescence. The fluorescent signal is then measured with the PMT or TDI camera as in the present invention. The green fluorescence is projected on the left area of the CCD and the fluorescence from the red laser excitation is projected on the right side of the CCD.

The magnification necessary for the image using a TDI camera depends on the resolution of the positional encode and the camera. The preferred encoder is a Heidenhein MT 2571 with a resolution of 0.2 microns. This encoder will give a TTL pulse every 0.2 microns, used to trigger the TDI camera and pulse results in one TDI row shift.

To achieve a square pixel image, the pixel size in the y-direction equals the size of a pixel in the x-direction. After each pulse the TDI row is moved with a corresponding distance lp = 13 microns on the CCD. This results in a magnification of 13/0.2 = 65 for this configuration. To use a different magnification, a counter is used. This counter gives 1 TTL pulse to the camera after 1,2,4,6 etc. pulses of the encoder. With this counter, the following magnifications can be used:

When the encoder is used, the set-up is constrained for a certain magnification and it is essential that the magnification is correct to ensure that the movement of the object and the shifting of the TDI rows occurs in synchrony. A mismatch of the magnification of the stage and the camera will result in a blurring of the image. When the magnification is too large or too small the image will be stretched in the forward or reverse direction, respectively. When the magnification is in synchrony, the speed of the x-y stage will vary, but will be limited by the maximum and minimum line rate of the camera. The minimum line rate of the camera is approximately 100 Hz, corresponding to a line time of 0.01 seconds and a total exposure time of 0.96 seconds for all TDI stages. Also if no encoder is used, the line rate and speed of the x-y stage can have a fixed value for a certain magnification.

Image resolution, particularly laterally, depends on the wavelength, focal distance, and entrance diameter, all limited by the diffraction limit of the objective. The one objective considered in the present invention is a Sony CD pick-up system (KSS-210ARP). The accurator of this CD system is used with a focal distance of f = 3.8 mm, a numerical aperture of N.A. = 0.45, and an opening entrance of D = 4 mm. The CD lens is designed to operate at a wavelength of 720 nm and, therefore, is expected to produce spherical and chromatic aberrations for other wavelengths which will reduce the resolution.

The resolution for the encoder is 0.2 microns for M = 65 and, with the shifting CCD rows, the illumination is always spread on two pixels, resulting in a resolution of 0.4 microns for the image. This resolution is higher than the optical limit and is limited by the optical resolution.

In the X-direction, a mismatch between the scanning direction and the TDI column results in a decrease of resolution. The movement of the cell should be parallel with the TDI columns. In the Y-direction, a mismatch of the magnification will also cause a reduced resolution with a blurring effect due to overlapping pixels.

The collection and illumination efficiency is determined by the light gathering power (N.A.) of the lens. In the present invention, the CD-lens is replaced with the achromat lens in the pickup accurator. The achromat lens has a focal distance of 7.5 mm and an entrance opening of 6.5 mm. The N.A. is approximately 0.27. The solid angle is 0.21 with the achromat lens, replacing the solid angle of 0.72 for the CD lens. The achromat is also used for the green laser in order to have the same focal distance. However, the solid angle of the achromat is much smaller than the solid angle in the CD lens. The solid angle is proportional to the intensity of the collected light, making the CD lens more efficient than the achromat. Although the achromat lens is limiting the sensitivity (by approximately a factor of 15 for both illumination and collection of photons), it is more convenient to implement with a second laser, making tracking and focusing of the system possible. An achromat with a higher N.A. would be preferable, with a numerical aperture (N.A. > 0.5). This results in a higher SNR and a higher resolution. The present invention is also considered with the use of a microscope objective, although this type of objective would mandate changing the tracking and focusing mechanism.

For imaging and spectral separation of the fluorescent emission, a prism or combination of filters can be used. Instead of using a dichroic mirror combination, a prism is inserted in front of the CCD. With a direct vision prism, the deflection angle depends on the wavelength and the deflection angle is almost parallel with the incoming ray of light. To obtain an image where the fluorescence is separated with no spectral overlap, the spectral separation must be large enough to separate more than the maximum cell size. For example, with a maximum cell size of 20 microns the emission of APC and PE is about 100 nm apart and the bandwidth of emission is approximately 10 nm, resulting in a pixel resolution of 2 microns in the X-direction. Thus, dyes with smaller emission spectrums are required for optical resolutions of 0.7 to around 1 micron. A dichroic/mirror combination can also be used whereby a high pass filter and an almost parallel mirror can be used to separate the fluorescent emission into two distinct areas. For example, the emission of PE excited with the green laser is reflected on the filter surface while the emission of APC is transmitted by the filter and reflected on the mirror.

Implementation of TDI imaging into the imaging systems like CellTracks® provides a method with good image resolution and an improved signal to noise compared to the analysis with the scanning mirror and the full frame CCD. The scan speed is also increased with the time to image one line under TDI imaging around 10 sec (at a speed of 1.5 mm/sec). Previous methods would only measure 1 cell within this time period. With the dual excitation laser, it is possible to image the fluorescent emission with more than one dye. Dyes that have the same absorption spectrums but different emission spectrums have an emission that is close together, demanding a narrow bandwidth of the filters to image the emission of these dyes.

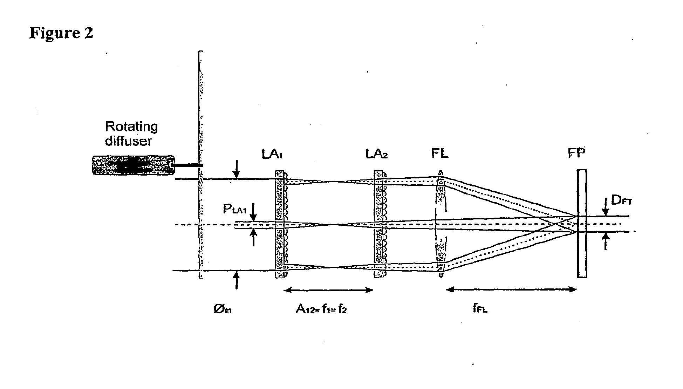

A homogeneous illumination of the sample is critical for a image cytometer to illuminate each cell with the same amount of light when it passes through the laser spot, while non-homogeneous illumination leads to a variation of the fluorecesence signal detected across the CCD. To prevent unnecessary bleaching and maximize the signal collection of the sample, the illumination area should also exactly match with the size of the CCD detector. To achieve this; a beam homogenizer (Suss-MicroOptics, Neuchatel,Switserland) is used as shown in figure 2. It consists out of a pair of micro lens arrays in combination with a rotating diffuser and is creating a square homogeneous profile.

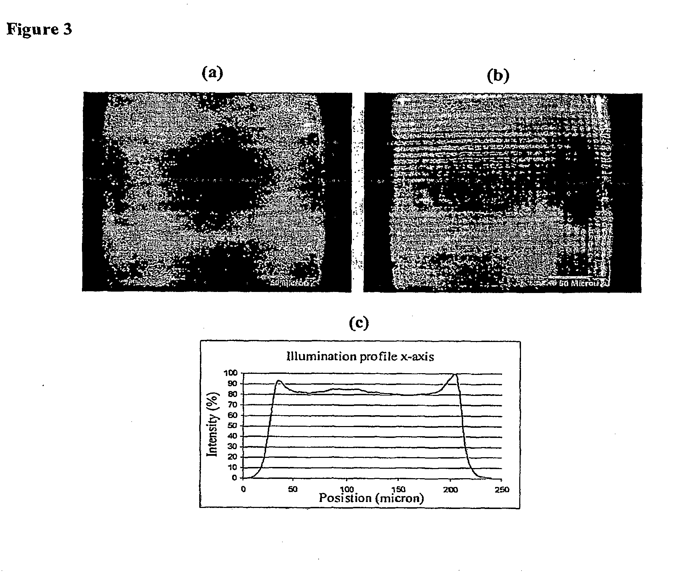

The rotating diffuser in front of the homogenizer is necessary to reduce the coherence length of the laser beams to prevent interference effects at the two micro lens arrays, as shown in figure 3. While the micro lens consist out of a periodic structure and acts like diffraction grating it will generate diffraction spots with a period $\Delta_{FP}$ of and a spot diameter $\delta_x$ in the Fourier plane.

$$\Delta_{FP} = \frac{f_{FL} \cdot \lambda}{P_{L1}} \quad (2.3) \qquad\qquad \delta_x = \frac{2 \cdot f_{FL} \cdot \lambda}{D_{FL}}$$

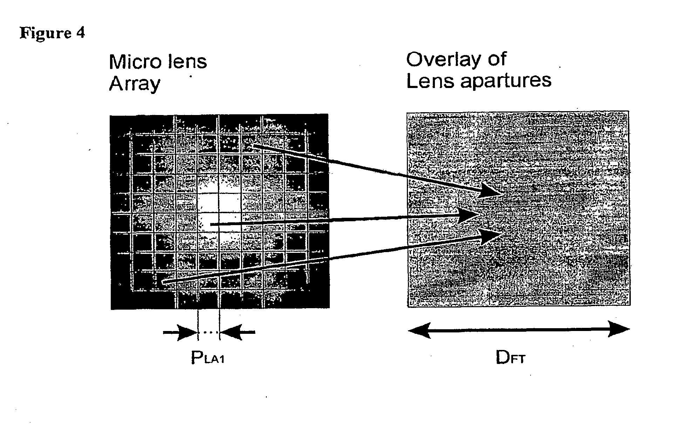

The first square microlens array $LA_1$ focuses the incoming beam at the next micro lens array $LA_2$, resulting in multiple point sources. All these quadratics lens apertures shown in figure 4 are imaged with the Fourier lens (microscope objective) and overlaid in the Fourier plan and form a quadratic flat top. The size of the flat top is defined by:

$$D_{FT} = \frac{P_{LA1} \cdot f_{FL}}{f_1 \cdot f_2} \cdot \left( (f_1 + f_2) - a_{12} \right)$$

Slightly varying the distance between the two micro lens arrays gives the possibility to adjust the size of the flat top, as long the lens apertures of the second lens array are not overfilled resulting in crosstalk between the lenslets and a less sharply defined border of the flat top profile. The flat top size can also be adjusted by using a different microscope objective; this is useful while the focal length of the objective is approximately inversely proportional with the magnification and results in a illumination area matching the size of the CCD detector when a microscope objective is used with a different magnification. As shown in figure 3 a homogenous layer of acridine orange is used to determine the beam profile perpendicular to the scanning direction. The resulting profile is approximately 200 x 200 $\mu$m (200 x 200 micron).

It will be apparent to those skilled in the art that the improved scanning and imaging system of the invention is not to be limited by the foregoing descriptions of preferred embodiments, and that the preferred embodiments of the invention which incorporate these improvements, as previously described, have also been found to enable the invention to be employed in many fields and applications to diagnosis of cells and to particulate target species in general. The following Examples illustrate specific of the invention, but are not thereby limited in scope.

**Claims**

1. A method of performing image cytometry, comprising:

   a. obtaining a sample containing rare cells from blood or other fluids;
   b. labeling said rare cells with a fluorophore;
   c. manipulating said rare cells towards a collection surface;
   d. illuminating said collected rare cells with a laser having a beam homogenizer with a rotating diffuser and microlens array, wherein said illuminating is homogenous across said rare cells; and
   e. imaging said rare cells by fluorescent microscopy wherein said fluorescent microscopy produces a homogeneous fluorescent image.

2. The method of claim 1 wherein said manipulating is by magnetically labeling with magnetic particles specific for said rare cells.

3. The method of claim 2 wherein magnetically labeled particles are aligned between Ni lines.

4. The method of claim 1 wherein said imaging is a composite analysis of sub-images.

5. The method of claim 4 wherein said sub-images are acquired by time-delay integration.

**Patentansprüche**

1. Verfahren zum Durchführen von Bildzytometrie, umfassend:

   a. Beschaffen einer Probe, die seltene Zellen aus Blut oder anderen Fluiden enthält;
   b. Markieren der seltenen Zellen mit einem Fluorophor;
   c. Manipulieren der seltenen Zellen hin zu einer Sammeloberfläche;
   d. Beleuchten der gesammelten seltenen Zellen mit einem Laser, der einen Strahlhomogenisator mit einem rotierenden Diffusor und einem Mikrolinsenarray aufweist, wobei die Beleuchtung über die seltenen Zellen homogen ist; und
   e. Abbilden der seltenen Zellen durch Fluoreszenzmikroskopie, wobei die Fluoreszenzmikroskopie ein homogenes Fluoreszenzbild erzeugt.

2. Verfahren gemäß Anspruch 1, wobei das Manipulieren durch magnetisches Markieren mit magnetischen Partikeln, die für die seltenen Zellen spezifisch sind, erfolgt.

3. Verfahren gemäß Anspruch 2, wobei magnetisch markierte Partikel zwischen Ni-Linien ausgerichtet werden.

4. Verfahren gemäß Anspruch 1, wobei die Bildgebung eine zusammengesetzte Analyse von Subbildern ist.

5. Verfahren gemäß Anspruch 4, wobei die Subbilder durch Zeitverzögerungsintegration aufgezeichnet werden.

**Revendications**

1. Procédé de réalisation d'une cytométrie d'image, comprenant :

   a. l'obtention d'un échantillon contenant de rares cellules de sang ou d'autres fluides ;
   b. le marquage desdites rares cellules avec un fluorophore ;
   c. la manipulation desdites rares cellules vers une surface de collecte ;
   d. l'éclairage desdites rares cellules collectées avec un laser ayant un homogénéisateur de faisceau avec un diffuseur rotatif et un réseau de microlentilles, ledit éclairage étant homogène d'un bout à l'autre desdites rares cellules ; et

e. l'imagerie desdites rares cellules par microscopie de fluorescence, ladite microscopie de fluorescence produisant une image fluorescente homogène.

2. Procédé selon la revendication 1 dans lequel ladite manipulation se fait par marquage magnétique avec des particules magnétiques spécifiques desdites rares cellules.

3. Procédé selon la revendication 2 dans lequel les particules marquées magnétiquement sont alignées entre des lignes de Ni.

4. Procédé selon la revendication 1 dans lequel ladite imagerie est une analyse composite de sous-images.

5. Procédé selon la revendication 4 dans lequel lesdites sous-images sont acquises par report-intégration.

**Figure 1**

**Figure 2**

Figure 3

**(a)**

**(b)**

**(c)**

Illumination profile x-axis

**Figure 4**

Micro lens Array

Overlay of Lens apartures

$P_{LA1}$

$D_{FT}$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6151405 A, Douglass
- US 5432054 A, Saunders
- US 10612144 B
- WO 0032293 A
- US 11344757 B

**Non-patent literature cited in the description**

- **RACILA et al.** *Proc. Nat. Acad. Sci.,* 1998, vol. 95, 4589-4594
- **TIBBE et al.** *Nature Biotech.,* 1999, vol. 17, 1210-13
- **RACILA et al.** *Proc. Nat. Acad. Sci.,* 1998, vol. 95, 4589-94
- **CORLE, TR.** Confocal Scanning Microscopy and Related Imaging Systems. Academic Press, 1995